# EUROPEAN PATENT APPLICATION

(11) **EP 3 640 318 A1**
(43) Date of publication of application: **22.04.2020**
(21) Application number: 18817507.9
(22) Date of filing: 13.06.2018
(51) Int. Cl.: C12M 1/00, A61F 2/02, A61L 27/14, A61L 27/20, A61L 27/28, A61L 27/38, A61L 27/40, A61L 27/50, A61L 27/52, A61L 27/54, A61L 27/56, A61K 9/50, A61K 9/52, A61K 35/12, A61K 35/28, A61K 35/39, A61K 47/30, A61L 27/34, A61P 5/50, A61P 43/00

(54) **CELL-SEALING DEVICE**

(30) Priority: 14.06.2017 JP 2017116832
(71) Applicant: Takeda Pharmaceutical Company Limited, Osaka-shi, Osaka 541-0045 (JP)
(72) Inventor: IWATA Hiroo, Osaka-shi Osaka 532-0024 (JP)
(74) Representative: Jones, Nicholas Andrew
(86) International application number: PCT/JP2018/022509
(87) International publication number: WO 2018/230588

(57) **Abstract**

As a cell-encapsulating device that may supply sufficient oxygen to cells in an implant part and therefore encapsulate a larger cell aggregate, there is provided a cell-encapsulating device comprising a plurality of capsule-form structures arrayed in two-dimensional directions in the same plane, wherein at least a part of outer shells of the capsule-form structures is formed from an oxygen-permeable membrane, and cells are encapsulated in the inside of the capsule-form structures.

## Description

### Technical Field

The present invention relates to a cell-encapsulating device and a method for producing the same.

### [Background Art]

Devices for cell implant therapy in which implant cells are encapsulated have been developed. Such a cell-encapsulating device can transfer implant cells low invasively and conveniently to a target site in the body. Also, the cell-encapsulating device has the advantage that the implant cells can be removed reliably and conveniently, together with the device, if the implant cells need to be taken out of the body.

Patent Literature 1 discloses an embedding assembly in which a porous boundary having a pore size capable of segregating implant cells from the immune response of a host tissue is formed between the host tissue and the implant cells in a chamber (see Patent Literature 1, claim 1). This device has one chamber for retaining cells and has a flat shape as a whole, and the chamber is described as "flat plate type" which is thin in the vertical direction and wide in the horizontal direction (see Patent Literature 1, Figures 8 and 9). Patent Literature 2 discloses an assembly for cell encapsulation comprising at least one chamber for encapsulating implant cells, wherein the assembly comprises a first seal at a peripheral edge of the assembly, and a second seal which effectively decreases the volume of the chamber but increases a device surface area (see Patent Literature 2, claim 16). This device also has a flat shape as a whole, and the chamber is described as "flat plate type" which is thin in the vertical direction and wide in the horizontal direction (see Patent Literature 2, Figure 1). For devices having such a flat plate-type chamber, also see Non Patent Literature 1.

Patent Literature 3 discloses "a 3-dimensional cell-encapsulating assembly comprising at least two cell chambers for encapsulating living cells, and a cell-free region along the longest axis separating the cell chambers, wherein the cell-free region is bent to form folds wherein the folds decrease the effective area of the assembly as compared to the assembly without the folds, thereby forming a 3-dimensional cell-encapsulating device" (see Patent Literature 3, claim 1). The individual chambers in this device are described as "flat plate type", and the flat plate-type chambers are connected through the folds and arrayed so that the whole device has a "blind-type" shape (see Patent Literature 3, Figures 3, 7, 15, 20, 57, and 62).

For enhancing the therapeutic effect of the cell-encapsulating device, it is desired that more cells, i.e., a larger cell aggregate, should be encapsulated in the device. On the other hand, for allowing implant cells to function in the living body, it is necessary to supply sufficient oxygen to the intra-device cells in an implant part, whereas a larger cell aggregate encapsulated therein is more prone to necrotize due to insufficient oxygen at the central part of the cell aggregate. In order to promote the supply of oxygen, reduction in the thickness of a porous membrane or the like may be possible. In this case, however, sufficient strength of the device is difficult to maintain.

### Citation List

### Patent Literature

Patent Literature 1: Japanese Patent Laid-Open No. 08-507949
Patent Literature 2: National Publication of International Patent Application No. 2012-508584
Patent Literature 3: National Publication of International Patent Application No. 2016-512022

### Non Patent Literature

Non Patent Literature 1: Ohgawara H, et. al., "Membrane immunoisolation of a diffusion chamber for bioartificial pancreas", Artif. Organs, 1998, 22(9):788-94
Non Patent Literature 2: Rezania A, et. Al., "Reversal of diabetes with insulin-producing cells derived in vitro from human pluripotent stem cells", Nat. Biotechnol., 2014, 32 (11) :1121-33
Non Patent Literature 3: Iwata H, et. al., Agarose for a bioartificial pancreas., J. Biomed. Mater. Res., 1992, 26(7):967-77

### Summary of Invention

### Technical Problem

A main object of the present invention is to provide a cell-encapsulating device that may supply sufficient oxygen to cells in an implant part and as a result, encapsulate a larger cell aggregate.

### Solution to Problem

In order to attain the object, the present invention provides the following [1] to [17]:
[1] A cell-encapsulating device comprising a plurality of capsule-form structures arrayed in two-dimensional directions in the same plane, wherein at least a part of outer shells of the capsule-form structures is formed from an oxygen-permeable membrane, and cells are encapsulated in the inside of the capsule-form structures.
[2] The cell-encapsulating device according to [1], wherein the oxygen-permeable membrane is a porous membrane or a hydrogel membrane.
[3] The cell-encapsulating device according to [1] or [2], wherein the cells are cells secreting a biologically active molecule.
[4] A method for producing a cell-encapsulating device, comprising the steps of:
   forming a plurality of depressions arrayed in two-dimensional directions on oxygen-permeable membranes;
   partially sealing the depressions to form capsule-form structures arrayed in two-dimensional directions in the same plane in which lumens of the capsule-form structures communicate with each other; and
   introducing cells to the capsule-form structures.
[5] A method for producing a cell-encapsulating device, comprising the steps of:
   forming a plurality of depressions arrayed in two-dimensional directions on oxygen-permeable membranes;
   introducing cells to the depressions; and
   laminating the concave surfaces of the oxygen-permeable membranes such that the depressions of the oxygen-permeable membranes are aligned with each other to form a capsule-form structures arrayed in two-dimensional directions in the same plane.
[6] A method for producing a cell-encapsulating device, comprising the steps of:
   forming a plurality of depressions arrayed in two-dimensional directions on oxygen-permeable membranes;
   introducing cells to the depressions;
   laminating the concave surfaces of the oxygen-permeable membranes such that the depressions of the oxygen-permeable membranes are aligned with each other to form a capsule-form structures arrayed in two-dimensional directions in the same plane; and
   allowing the cells to proliferate in the capsule-form structures.
[7] The method for producing a cell-encapsulating device according to any of [4] to [6], wherein the depressions are formed in the oxygen-permeable membrane by a heat setting method or a vacuum molding method.
[8] The method for producing a cell-encapsulating device according to any of [4] to [7], wherein the oxygen-permeable membrane is a porous membrane or a hydrogel membrane.
[9] The method for producing a cell-encapsulating device according to any of [4] to [8], wherein the cells are cells secreting a biologically active molecule.
[10] A method for producing a cell-encapsulating device, comprising the steps of:
   arraying, in two-dimensional directions in the same plane, a plurality of capsule-form structures in which at least a part of outer shells of the capsule-form structures is formed from an oxygen-permeable membrane, and cells are encapsulated in the inside of the capsule-form structures;
   contacting the plurality of capsule-form structures thus arrayed with a base material coated with an oxygen-permeable membrane-forming solution; and
   solidifying the oxygen-permeable membrane-forming solution.
[11] The method for producing a cell-encapsulating device according to [10], wherein the oxygen-permeable membrane is a porous membrane or a hydrogel membrane.
[12] The method for producing a cell-encapsulating device according to [10] or [11], wherein the cells are cells secreting a biologically active molecule.
[13] A method for producing a cell-encapsulating device, comprising the steps of:
   receiving cells into a plurality of depressions arrayed in two-dimensional directions on a substrate and isolated from each other with partition walls;
   introducing an oxygen-permeable membrane-forming solution onto the depression-formed surface of the substrate; and
   solidifying the oxygen-permeable membrane-forming solution, and separating an oxygen-permeable membrane, together with the cells, from the substrate.
[14] A method for producing a cell-encapsulating device, comprising the steps of:
   receiving cells into a plurality of depressions arrayed in two-dimensional directions on a substrate and isolated from each other with partition walls;
   allowing the cells to proliferate in the depressions;
   introducing an oxygen-permeable membrane-forming solution onto the depression-formed surface of the substrate; and
   solidifying the oxygen-permeable membrane-forming solution, and separating an oxygen-permeable membrane, together with the cells, from the substrate.
[15] The method for producing a cell-encapsulating device according to [13] or [14], wherein the specific gravity of the oxygen-permeable membrane-forming solution is determined according to the specific gravity of the cells or an aggregate thereof.
[16] The method for producing a cell-encapsulating device according to any of [13] to [15], wherein the cells are cells secreting a biologically active molecule.
[17] A medicament comprising a cell-encapsulating device according to any of [1] to [3].

In the present specification, the term "substantially" or "essentially" refers to a value equal to or more than 90%, preferably equal to or more than 95%, 96%, 97%, 98%, or 99%, of a reference value. For example, the term "substantially identical" or "essentially identical" means identity of 90% or higher, preferably 95%, 96%, 97%, 98%, or 99% or higher, to the reference value, and the term "substantially free of" or "essentially free of" means that a certain substance is contained at a content of not more than 5% or is undetectable.

In the present specification, the term "comprise(s)" or "comprising" refers to inclusion of the element(s) following the word without limitations thereto. Thus, this suggests inclusion of the element(s) following the word, but does not suggest exclusion of any other element.

In the present specification, the term "consist(s) of" or "consisting of" means inclusion of every element following the term and a limitation thereto. Thus, the term "consist(s) of" or "consisting of" indicates that the enumerated element(s) is required or essential, and substantially no other elements exist. The term "consist(s) essentially of" or "consisting essentially of" means inclusion of any element following the term and a limitation of other elements to those influencing neither the activity nor the effect defined in the present disclosure as to the element following the term. Thus, the term "consist(s) essentially of" or "consisting essentially of" indicates that the enumerated element(s) is required or essential, but other elements are optional and may or may not exist depending on whether to affect the activity or the effect of the enumerated element(s).

In the present specification, the term "*ex vivo*" is generally used to refer to an experiment or measurement conducted in living tissues in an artificial environment outside the living body, such as cultured tissues and cultured cells. The tissues or cells used may be frozen for preservation or may be thawed for subsequent treatment outside the living body. The term "*in vitro*" is used when a tissue culture experiment of living cells or living tissues is conducted for several consecutive days or longer. The term "*in vitro*" may be used interchangeably with *"ex vivo*"*.* By contrast, the term "*in vivo*" is generally used to refer to a phenomenon occurring in the living body, such as proliferation of cells.

In the present specification, the term "culture" refers to maintenance, proliferation (growth), and/or differentiation of cells in an *in vitro* environment. The term "culturing" means that cells are maintained, allowed to proliferate (grow), and/or differentiated outside a tissue or the living body, for example, in a cell culture dish or flask.

### Advantageous Effects of Invention

The present invention provides a cell-encapsulating device that may supply sufficient oxygen to cells in an implant part and as a result, encapsulate a larger cell aggregate.

### Brief Description of Drawings

[Figure 1] Figure 1 is a diagram showing a first embodiment of the cell-encapsulating device of the present invention. Figure 1(A) is a top perspective view and a partially enlarged sectional view, and Figure 1(B) is a cross-sectional view.
[Figure 2] Figure 2 is a diagram showing a second embodiment of the cell-encapsulating device of the present invention.
[Figure 3] Figure 3 is a diagram showing a third embodiment of the cell-encapsulating device of the present invention.
[Figure 4] Figure 4 is a diagram showing a fourth embodiment of the cell-encapsulating device of the present invention. Figure 4(A) is a top perspective view, and Figure 4(B) is a cross-sectional view.
[Figure 5] Figure 5 is a diagram showing a first embodiment of the method for producing a cell-encapsulating device according to the present invention.
[Figure 6] Figure 6 is a diagram showing an exemplary modification of the method for producing a cell-encapsulating device according to the first embodiment.
[Figure 7] Figure 7 is a diagram showing a second embodiment of the method for producing a cell-encapsulating device according to the present invention.
[Figure 8] Figure 8 is a diagram showing the configuration of a communication path that connects capsule-form structures.
[Figure 9] Figure 9 is a diagram showing a third embodiment of the method for producing a cell-encapsulating device according to the present invention.
[Figure 10] Figure 10 is a diagram showing a fourth embodiment of the method for producing a cell-encapsulating device according to the present invention.

### Description of Embodiments

Hereinafter, the preferred modes for carrying out the present invention will be described with reference to the drawings. The embodiments described below are given for merely illustrating typical embodiments of the present invention and should not be construed as a limitation of the scope of the present invention.

### 1. Cell-encapsulating device

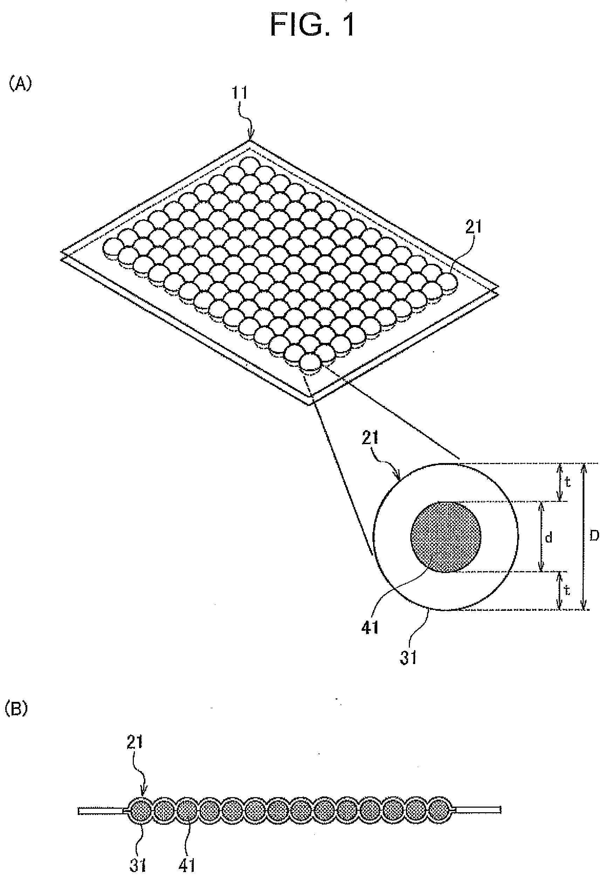

Figure 1 shows a first embodiment of the cell-encapsulating device of the present invention. Cell-encapsulating device 11 comprises a plurality of capsule-form structures 21 arrayed in two-dimensional directions in the same plane.

In the present invention, the capsule-form structures mean structures that are composed of a spherical or substantially spherical outer shell or a hemispherical or substantially hemispherical outer shell and have a space (lumen) within the outer shell.

The term "substantially spherical" means that the ratio between a diameter in any one direction of a structure and a diameter in a direction orthogonal thereto is 0.7 to 1.3, particularly, 0.8 to 1.2. The term "substantially hemispherical" is either of two structures obtained by cutting a spherical or substantially spherical structure in any one plane.

In the cell-encapsulating device 11, each capsule-form structure 21 is composed of spherical or substantially spherical outer shell 31 and has lumen 41 within the outer shell 31. In the present specification, when the capsule-form structures 21 have a substantially spherical shape, the outside diameter of the outer shell 31 and the inside diameter of the lumen 41 mean their respective major axes.

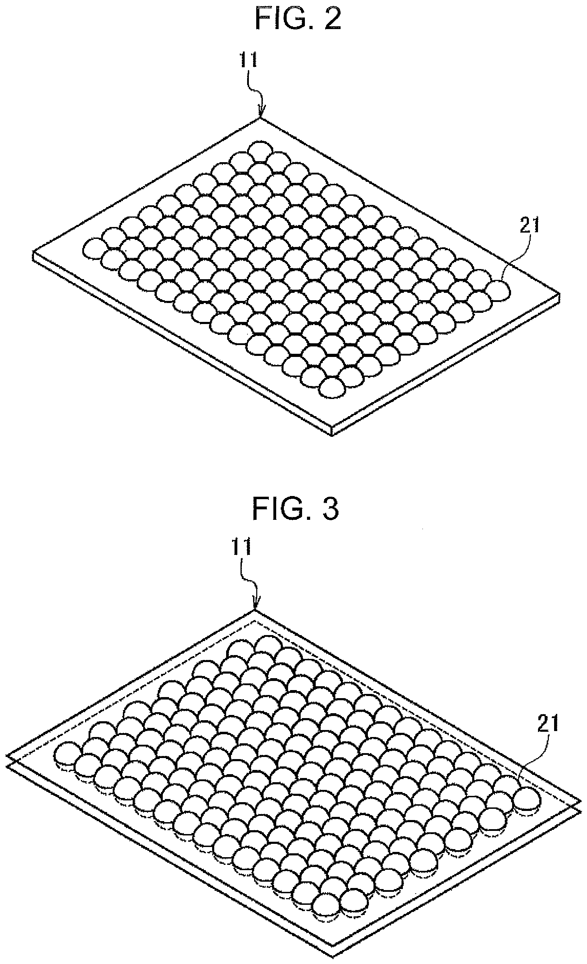

In the cell-encapsulating device according to the present invention, the capsule-form structures may be hemispherical or substantially hemispherical, as shown in Figure 2. Hereinafter, the terms "spherical", "substantially spherical", "hemispherical" and "substantially hemispherical" are also collectively referred to as simply "substantially spherical".

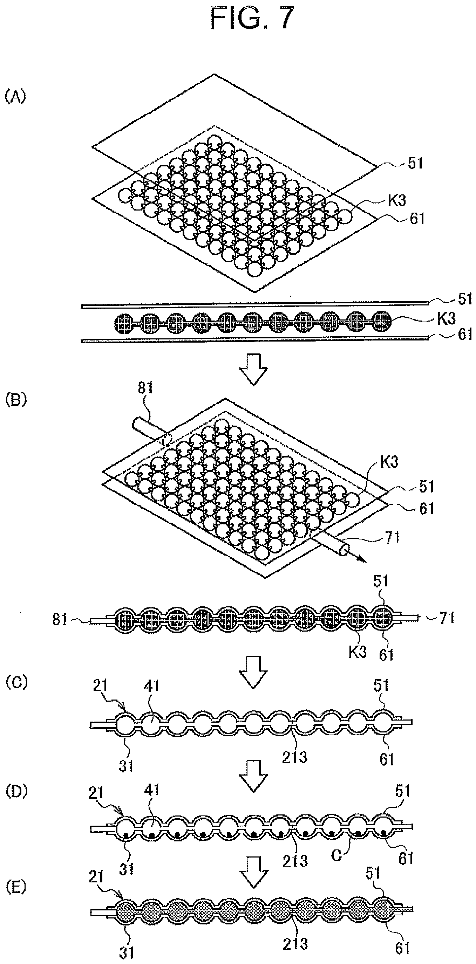

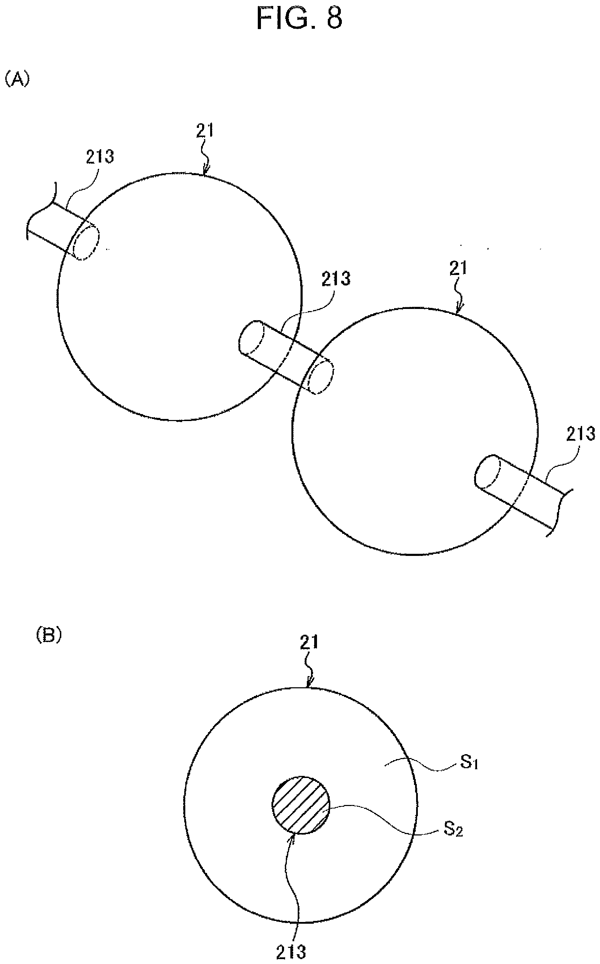

The lumens of the capsule-form structures may be enclosed spaces sequestered from the external space of the device through the outer shells. Alternatively, the lumens of adjacent capsule-form structures may communicate with each other (see Figures 7 and 8 mentioned later).

The outer shells 31 of the capsule-form structures 21 assume the contours of the capsule-form structures 21, and at least a portion thereof is formed from an oxygen-permeable membrane. Oxygen is thereby supplied through the oxygen-permeable membrane to cells encapsulated in the lumens 41 in an implant part. The oxygen-permeable membrane needs to have permeability at least to oxygen and may have permeability to a gas other than oxygen, and a nutrient necessary for cell survival as long as the cells encapsulated in the lumens 41 are not leaked out of the device of the present invention. The oxygen-permeable membrane further has permeability to a substance produced by the cells encapsulated in the lumens 41. The substance produced by the cells encapsulated in the lumens 41 is thereby released to the implant part through the oxygen-permeable membrane.

Examples of the gas other than oxygen include carbon dioxide and nitrogen.

Examples of the nutrient necessary for cell survival include saccharides, amino acids, lipids, vitamins and minerals.

On the other hand, the oxygen-permeable membrane needs to block passage of cells of a host and, preferably, an antibody of the host, for functioning as a partition wall between the cells encapsulated in the lumens 41 and the cells of the host. This may avoid eliminating or inactivating implant cells by immune response from host cells.

The oxygen-permeable membrane can be a porous membrane.

The pore size of the porous membrane is set to 10 to 5000 nm, preferably 50 to 1000 nm, more preferably 100 to 500 nm, for permeability to oxygen, a nutrient and a substance produced by the cells. It is preferred that a plurality of pores carried by the oxygen-permeable membrane should be uniformly dispersed throughout the oxygen-permeable membrane such that necessary amounts of oxygen, a nutrient and a substance produced by the cells can permeate the oxygen-permeable membrane as a whole.

Examples of the material of the porous membrane include materials having thermoplasticity (polyvinylidene fluoride, acrylonitrile-vinyl chloride copolymers, polyvinyl chloride, nylon, polysulfone, polyethersulfone, ethylene-vinyl alcohol copolymers, polyester polymer alloys, polypropylene, oriented polypropylene, ion-track etched polyester and ion-track etched polycarbonate), and materials having no thermoplasticity (expanded polytetrafluoroethylene (EPTFE), regenerated cellulose, cellulose acetate and mixed cellulose ester).

The oxygen-permeable membrane may be a hydrogel membrane that swells by water.

Examples of the material of the hydrogel membrane include: natural polymers such as agarose, alginic acid, hyaluronic acid, cellulose and gelatin; and chemically or physically cross-linked forms of synthetic polymers such as polyvinyl alcohol, polyacrylamide, polyacrylic acid, polymethacrylic acid, polyisopropylacrylamide, poly-2-hydroxyethyl methacrylate, poly-2-hydroxyethyl acrylate and polyvinylpyrrolidone, and copolymers of each monomer.

The oxygen-permeable membrane may be a commercially available product or may be prepared from a solution that is solidified to form an oxygen-permeable membrane by a method known per se (oxygen-permeable membrane-forming solution).

Cells are encapsulated in the lumens 41. Examples of the cells include cells secreting biologically active molecules such as hormones (insulin, etc.) and cytokines (e.g., beta cells of the pancreas and progenitor cells thereof).

These cells can be *ex vivo* cells separated from a donor or may be cells cultured *in vitro.* The cells cultured *in vitro* can be, for example, pluripotent stem cells such as embryonic stem cells (ES cells) or induced pluripotent stem cells, or multipotent stem cells such as mesenchymal stem cells, or can be cells obtained by inducing the differentiation of these cells.

In this context, the "pluripotent stem cells" refer to embryonic stem cells (ES cells) and cells potentially having pluripotency similar to that of ES cells, i.e., the ability to differentiate into various tissues (all of the endodermal, mesodermal, and ectodermal tissues) in the living body. Examples of the cells having pluripotency similar to that of ES cells include "induced pluripotent stem cells" (in the present specification, also referred to as "iPS cells").

Examples of the "ES cells" that may be utilized include mouse ES cells including various mouse ES cell lines established by inGenious Targeting Laboratory, Inc., RIKEN, and the like, and human ES cells including various human ES cell lines established by Thomson (University of Wisconsin, USA), NIH (USA), RIKEN, Kyoto University, and Cellartis AB. Examples of the human ES cell lines that can be utilized include CHB-1 to CHB-12, RUES1, RUES2, HUES1 to HUES28 lines from NIH, H1 and H9 lines from WisCell Research Institute Inc., and KhES-1, KhES-2, KhES-3, KhES-4, KhES-5, SSES1, SSES2, and SSES3 lines from RIKEN.

The "induced pluripotent stem cells" refers to cells that are obtained by reprograming mammalian somatic cells or undifferentiated stem cells by introducing particular factors (nuclear reprogramming factors). At present, there are various "induced pluripotent stem cells", and iPS cells established by Yamanaka, et al. by introducing four factors Oct3/4, Sox2, Klf4, and c-Myc into mouse fibroblasts (Takahashi K, Yamanaka S., Cell, (2006) 126: 663-676) as well as human cell-derived iPS cells established by introducing similar four factors into human fibroblasts (Takahashi K, Yamanaka S., et al., Cell, (2007) 131: 861-872.), Nanog-iPS cells established by sorting cells with Nanog expression as an index after introduction of the four factors (Okita, K., Ichisaka, T., and Yamanaka, S. (2007). Nature 448, 313-317.), iPS cells prepared by a c-Myc-free method (Nakagawa M, Yamanaka S., et al., Nature Biotechnology, (2008) 26, 101-106), and iPS cells established by introducing six factors by a virus-free method (Okita K et al., Nat. Methods 2011 May; 8 (5): 409-12; and Okita K et al., Stem Cells. 31 (3) 458-66) can also be used. Also, induced pluripotent stem cells established by introducing four factors OCT3/4, SOX2, NANOG, and LIN28 prepared by Thomson et al. (Yu J., Thomson JA. et al., Science (2007) 318: 1917-1920), induced pluripotent stem cells prepared by Daley et al. (Park IH, Daley GQ.et al., Nature (2007) 451: 141-146), induced pluripotent stem cells produced by Sakurada et al. (Japanese Patent Laid-Open No. 2008-307007), and the like may be used.

In addition, any of induced pluripotent stem cells known in the art, described in all published papers (e.g., Shi Y., Ding S., et al., Cell Stem Cell, (2008) Vol. 3, Issue 5, 568-574;, Kim JB., Scholer HR., et al., Nature, (2008) 454, 646-650; and Huangfu D., Melton, DA., et al., Nature Biotechnology, (2008) 26, No 7, 795-797) or patents (e.g., Japanese Patent Laid-Open No. 2008-307007, Japanese Patent Laid-Open No. 2008-283972, US2008-2336610, US2009-047263, WO2007-069666, WO2008-118220, WO2008-124133, WO2008-151058, WO2009-006930, WO2009-006997, and WO2009-007852) can be used.

Readily available induced pluripotent stem cell lines include various iPS cell lines established by NIH, RIKEN, Kyoto University, and the like. Examples thereof include human iPS cell lines such as HiPS-RIKEN-lA, HiPS-RIKEN-2A, HiPS-RIKEN-12A, and Nips-B2 lines from RIKEN, and 253G1, 201B7, 409B2, 454E2, 606A1, 610B1, and 648A1 from Kyoto University.

The "mesenchymal stem cells" are multipotent stem cells that may differentiate into mesenchymal cells including osteoblasts, myocytes, chondrocytes, and adipocytes. In the present invention, the mesenchymal stem cells may be cells isolated from a living tissue or may be cells derived from ES cells or iPS cells. Markers specific for the mesenchymal stem cells are described in, for example, Vasileios Karantalis and Joshua M. Hare, Circ Res. 2015 April 10; 116 (8): 1413-1430, and Imran Ullah, et al., Biosci. Rep. (2015), 35/art:e00191, though the markers are not limited to those described therein.

The cells to be encapsulated in the lumens 41 can be of one type or two or more types. One or two or more cells may be encapsulated in each of the lumens 41. Two or more cells to be encapsulated may be dispersed cells or may be an aggregated cell mass.

Examples of the substance produced by these cells include biologically active substances including: hormones such as insulin, glucagon, growth hormone, parathormone and steroid; and neurotransmitters such as dopamine, serotonin, adrenaline and noradrenaline.

Diameter (inside diameter) d of the lumens 41 of the capsule-form structures 21 is set to {(ρ / (2 + ρ)}^{1/2}rₛ₀₀ or smaller (wherein "rₛ₀₀" represents the maximum radius of a spherical cell aggregate that does not cause cell necrosis when the cell aggregate is directly implanted in the living body; and "ρ" represents the ratio of an oxygen diffusion constant (D₁) within the oxygen-permeable membrane (outer shells 31) to an oxygen diffusion constant (D₀) within the cell aggregate (D₁/D₀)).

Thickness t of the outer shells 31 is not limited by any means as long as the inside diameter d of the lumens 41 falls within the numerical range described above. The thickness t can be, for example, 0.1 µm or larger and may be set to preferably 1 µm or larger, more preferably 10 µm or larger.

Outside diameter D of the capsule-form structures 21 is defined by the inside diameter d of the lumens 41 and the thickness t of the outer shells 31.

The capsule-form structures 21 are arrayed in two-dimensional directions in the same plane.

The number of capsule-form structures 21 arranged is not particularly limited and may be arbitrarily set according to the type of implant cells, an implant site and the purpose of implantation, etc.

The number of capsule-form structures 21 arranged is set to, for example, 10 to 100000 long × 10 to 10000 wide, preferably 100 to 10000 long × 10 to 10000 wide, more preferably 100 to 1000 long × 100 to 10000 wide.

However, an array of 1 long × 2 or more wide (array in a one-dimensional direction) is not excluded from the array pattern of the capsule-form structures 21 in the cell-encapsulating device according to the present invention. A plurality of cell-encapsulating devices 11 may be stacked and placed so that the capsule-form structures 21 are arrayed in three-dimensional directions. The array pattern of the capsule-form structures 21 in the cell-encapsulating device according to the present invention is not particularly limited as long as the flow of body fluid is secured around the individual capsule-form structures 21 in an implant part and an oxygen concentration is maintained.

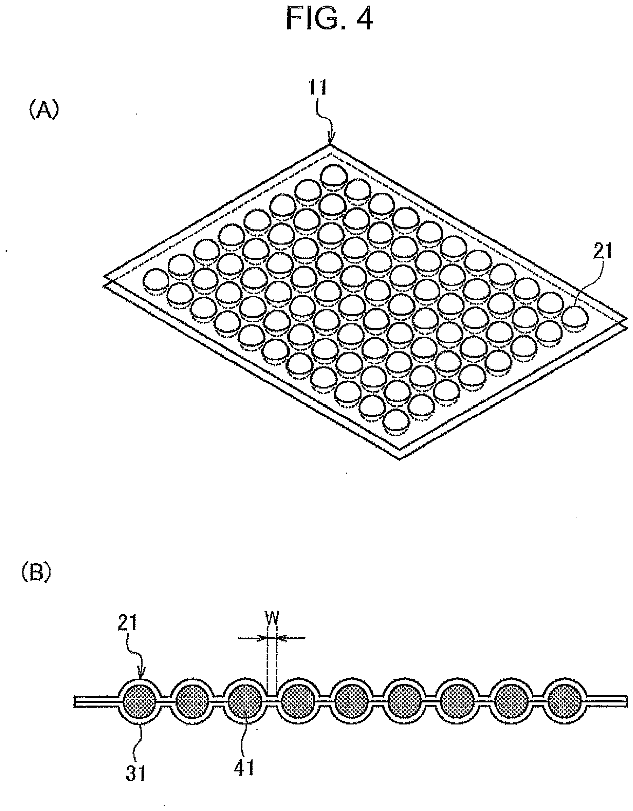

In the present invention, for arraying many capsule-form structures in the same plane, it is preferred to densely array the capsule-form structures so as to attain the smallest distance therebetween. However, the capsule-form structures may be arrayed at a predetermined distance from each other. Specifically, the capsule-form structures 21 are preferably placed by the closest packing as shown in Figure 3, but may be arrayed at distance W from each other as shown in Figure 4. The cell-encapsulating device 11 may have a planar region between the capsule-form structures 21.

Since the capsule-form structures according to the present invention have a substantially spherical contour, oxygen supply to the lumens from the outside may be efficiently performed in an implant part. Unlike a "flat plate-type" chamber or a "blind-type" chamber, which conventional cell-encapsulating devices have and a cell aggregate is encapsulated in a flat plate form, the thickness of the membrane for use in encapsulating is therefore not limited. Thus, it can be expected that the cells or the cell aggregate (or a part of the cell aggregate) encapsulated in the lumens is less likely to necrotize. Accordingly, the cell-encapsulating device according to the present invention eliminates the need of using a thin and robust oxygen-permeable membrane, which is generally difficult to obtain, and drastically improves the freedom of choice of the membrane.

For efficiently performing oxygen supply to the lumens from the outside in an implant part, it is preferred that the capsule-form structures should each independently have a substantially spherical contour and be arrayed without fusing their respective curved surfaces. However, the fusion between the capsule-form structures is not completely excluded, and the capsule-form structures may be in point contact or in contact at a small area with each other. In this embodiment, the lumens of the capsule-form structures may or may not communicate with each other.

The cell-encapsulating device obtained according to the present invention is useful as a cell medicament by implanting the cell-encapsulating device in a state containing cells or by encapsulating cells after implantation. Particularly, the cell-encapsulating device can be used as a medicament comprising cells secreting a biologically active molecule such as a hormone (insulin, etc.) or cytokine (e.g., beta cells of the pancreas and progenitor cells thereof) in the treatment of diabetes mellitus (type 1 diabetes mellitus, type 2 diabetes mellitus, etc.).

### 2. Method for producing cell-encapsulating device

### (1) Production method I

A first embodiment of the method for producing a cell-encapsulating device according to the present invention comprises the following steps (1-2) and (AI-3) and optionally further comprises steps (I-1) and (I-4):
(I-1) forming a plurality of depressions arrayed in two-dimensional directions on oxygen-permeable membranes;
(I-2) introducing cells to the depressions;
(I-3) laminating the concave surfaces of the oxygen-permeable membranes such that the depressions of the oxygen-permeable membranes are aligned with each other to form a capsule-form structures arrayed in two-dimensional directions in the same plane; and
(I-4) allowing the cells to proliferate in the capsule-form structures.

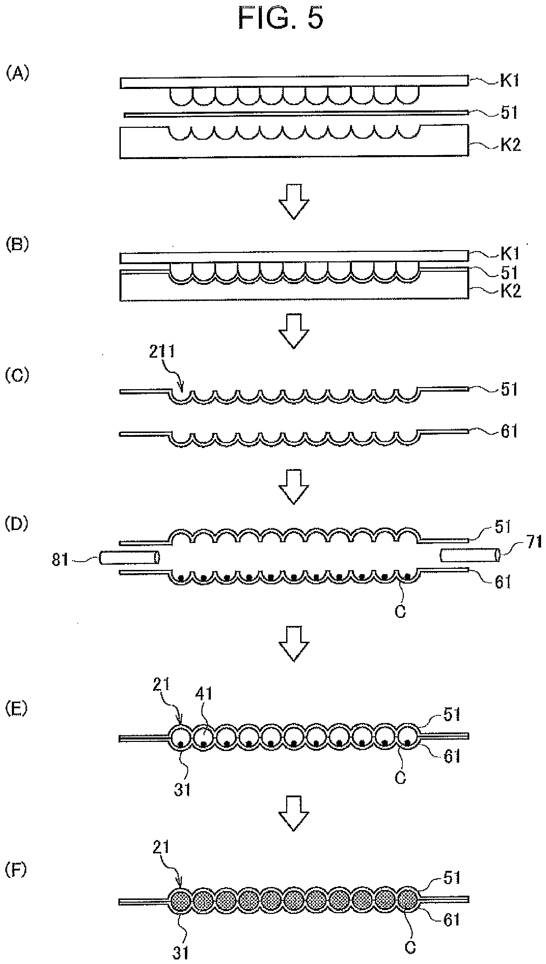

Hereinafter, these steps will be described in order with reference to Figure 5.

### (I-1) Depression formation step

In this step, a plurality of depressions 211 (see Figure C) arrayed in two-dimensional directions on each of two oxygen-permeable membranes 51 and 61 are formed. The depressions 211 have a shape appropriate for outer shells 31 of capsule-form structures 21 (see Figure E), and the size thereof may be appropriately set in consideration of the diameter (inside diameter) of lumens 41 of the capsule-form structures 21.

Specifically, male mold K1 and female mold K2 having shapes corresponding to the depressions 211 are first provided (see Figure A). Next, oxygen-permeable membrane 51 made of a thermoplastic material such as polyvinylidene fluoride, acrylonitrile-vinyl chloride copolymers, polyvinyl chloride, nylon, polysulfone, polyethersulfone, ethylene-vinyl alcohol copolymers, polyester polymer alloys, polypropylene, oriented polypropylene, ion-track etched polyester or ion-track etched polycarbonate is molded by a heat setting method using the male mold K1 and the female mold K2 (see Figure B). Likewise, another oxygen-permeable membrane 61 is also molded. The thus-molded oxygen-permeable membranes 51 and 61 separated from the molds are shown in Figure C. In this respect, the thickness of the oxygen-permeable membranes 51 and 61 may be appropriately set in consideration of the thickness of the outer shells 31 of the capsule-form structures 21. In the case of using a material having no thermoplasticity, such as expanded polytetrafluoroethylene (EPTFE), regenerated cellulose, cellulose acetate or mixed cellulose ester, as the oxygen-permeable membranes 51 and 61, a plasticizer or the like is appropriately added to the material, which is then molded by a heat setting method.

The oxygen-permeable membranes 51 and 61 thus molded may be sterilized, if necessary.

### (1-2) Cell introduction step

In this step, cells C are introduced to the depressions 211 in one of the oxygen-permeable membranes 51 and 61 (see Figure D).

The introduction of the cells C can adopt, for example, a method which involves placing the oxygen-permeable membranes 51 and 61 such that their concave surfaces face each other to prepare a bag form, inserting inlet tube 71 to the space formed between these membranes, and introducing a cell suspension. It is preferred to insert outlet tube 81 for discharging the cell suspension from the space formed between the oxygen-permeable membranes 51 and 61, to the opposite side of the inlet tube 71.

### (1-3) Lamination step

In this step, the concave surfaces of the oxygen-permeable membranes 51 and 61 are laminated such that the depressions 211 of the oxygen-permeable membranes 51 and 61 are aligned with each other. The depressions 211 are thereby encapsulated to form capsule-form structures 21 arrayed in two-dimensional directions in the same plane, thereby obtaining a cell-encapsulating device (see Figure E). In this respect, the inlet tube 71 and the outlet tube 81 can be removed.

### (1-4) Cell proliferation step

In this step, the cells C are allowed to proliferate in the lumens 41 of the capsule-form structures 21, if necessary.

The proliferation of the cells C can be performed, for example, by dipping the cell-encapsulating device obtained after the lamination step in a culture medium suitable for the proliferation of the cells C, and placing the culture medium in an atmosphere suitable for cell proliferation. The proliferation of the cells forms cell aggregates of the cells C in the lumens 41.

The culture of the cells can be performed by the application of conventionally known conditions according to the type of the cells. For example, the culture of pancreatic progenitor cells can adopt a method described in Non Patent Literature 2.

In this step, the cells C before proliferation or after proliferation may be further induced to differentiate, if necessary.

The induction of differentiation can be performed by the application of conventionally known conditions according to the types of starting cells and differentiated cells. For example, the induction of differentiation of pancreatic progenitor cells into beta cells of the pancreas can adopt a method described in Non Patent Literature 2.

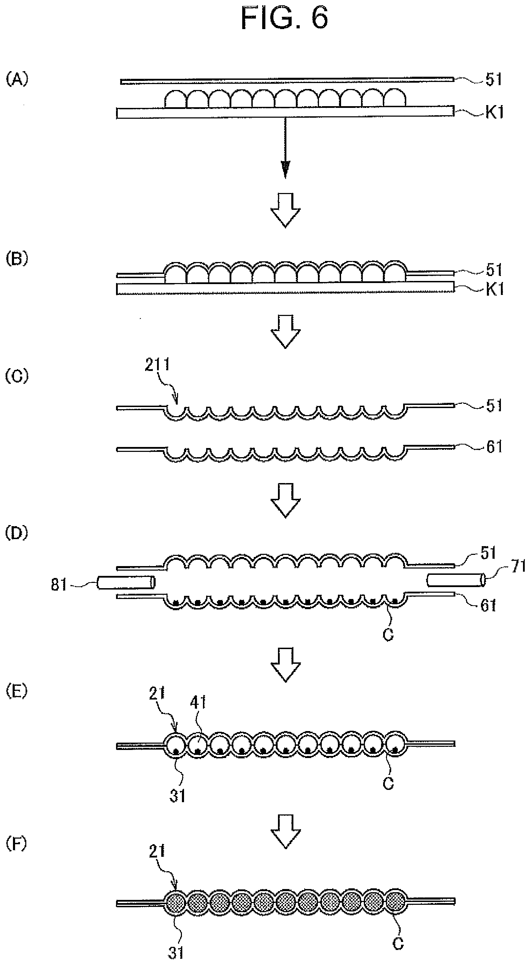

Figure 6 shows an exemplary modification of the method for producing a cell-encapsulating device according to the first embodiment mentioned above, and this modified method differs from the method mentioned above only in the depression formation step (I-1). In the present invention, a vacuum molding method may be used instead of the heat setting method mentioned above in the depression formation step (I-1).

Specifically, in the production method shown in Figure 6, the male mold K1 mentioned above is used alone in the depression formation step (I-1). Oxygen-permeable membrane 51 is positioned on the male mold K1 (see Figure A), and the resultant is placed in vacuum so that the oxygen-permeable membrane 51 is pressure-bonded to the male mold K1 to form depressions 211 in the oxygen-permeable membrane 51 (see Figure B). The vacuum molding may be performed by using the female mold K2 alone and pressure-bonding the oxygen-permeable membrane 51 thereto in vacuum, as a matter of course.

In the case of using a porous membrane as the oxygen-permeable membranes 51 and 61, the pores in the membrane may be temporality closed by coating with a removable polymer or a solution thereof in order to enhance a molding effect under vacuum conditions. After molding, the polymer is removed from the membrane using a solvent.

Examples of the polymer include polyvinyl alcohol.

### (2) Production method II

A second embodiment of the method for producing a cell-encapsulating device according to the present invention comprises the following steps (11-2) and (AII-3) and optionally further comprises steps (II-1) and (II-4) :
(II-1) forming a plurality of depressions arrayed in two-dimensional directions on oxygen-permeable membranes;
(II-2) partially sealing the depressions to form capsule-form structures arrayed in two-dimensional directions in the same plane in which lumens of the capsule-form structures communicate with each other;
(II-3) introducing cells to the capsule-form structures; and
(II-4) allowing the cells to proliferate in the capsule-form structures.

Hereinafter, these steps will be described in order with reference to Figure 7.

### (II-1) Depression formation step

In this step, a plurality of depressions arrayed in two-dimensional directions on each of two oxygen-permeable membranes 51 and 61 are formed. In the depression formation step (I-1) of the production method I mentioned above, the mode of forming depressions 211 by applying both male mold K1 and female mold K2 to each of the oxygen-permeable membranes 51 and 61 is described. By contrast, in this step, depressions are formed in both oxygen-permeable membranes 51 and 61 at once using resin mold K3.

Specifically, resin mold K3 having a shape corresponding to the depressions is first provided (see Figure A). The resin mold K3 has a shape corresponding to communication paths 213 between capsule-form structures 21 mentioned later, in addition to the shape corresponding to the depressions.

Depressions are formed in the oxygen-permeable membranes 51 and 61 by heat setting or vacuum molding in a state where the resin mold K3 is positioned between the oxygen-permeable membranes 51 and 61 (see Figure B). In this respect, it is preferred to insert inlet tube 71 for introducing a solvent (which dissolves the resin mold K3) and a cell suspension to between the oxygen-permeable membranes 51 and 61, and to insert outlet tube 81 for discharging the dissolved resin mold K3 and the cell suspension to the opposite side of the inlet tube 71.

The thickness of the oxygen-permeable membranes 51 and 61 may be appropriately set in consideration of the thickness of the outer shells 31 of the capsule-form structures 21. The oxygen-permeable membranes 51 and 61 thus molded may be sterilized, if necessary.

### (II-2) Encapsulating step

In this step, the depressions are partially sealed to form capsule-form structures 21 arrayed in two-dimensional directions in the same plane in which lumens 41 of the capsule-form structures 21 communicate with each other.

The oxygen-permeable membranes 51 and 61 and the resin mold 3 after the step (II-1) are dipped in a solvent to dissolve and remove the resin mold 3, thereby obtaining capsule-form structures 21 having lumens 41. In order to enable removal with a solvent, for example, polystyrene, methyl methacrylate or polycarbonate is used as the material of the resin mold K3. Toluene, benzene, chloroform, or the like is used as the solvent.

Since the oxygen-permeable membranes 51 and 61 after the removal of the resin mold K3 have a shape that is derived from the shape of the resin mold 3 and corresponds to the communication paths 213 of the capsule-form structures 21 (see Figure C), the concave surfaces of these membranes may be laminated except for the moiety of the communication paths 213 to obtain capsule-form structures 21 with their lumens 41 communicating with each other via the communication paths 213 (see Figure C). The capsule-form structures 21 communicate with adjacent other capsule-form structures 21 only via the communication paths 213, while the other moieties are encapsulated (also see Figure 8).

The lower limit of the inside diameter of the communication paths 213 is not particularly limited as long as a cell suspension introduced in the subsequent cell introduction step (11-3) is circulatable. The lower limit of the inside diameter of the communication paths 213 is, for example, on the order of 10 to 30 µm which is equivalent to the diameter of cells. On the other hand, the upper limit of the inside diameter of the communication paths 213 (and the outside diameter of the communication paths 213 determined depending thereon) is set such that the percentage of an area to be subjected to the connection of the communication paths 213 (in Figure 8(B), see reference symbol S₂) with respect to the outside surface area of the capsule-form structures 21 (Figure 8(B), see reference symbol S₁) is 20% or less. This percentage is preferably 15% or less or 10% or less, more preferably 5% or less or 3% or less, particularly preferably 2% or 1% or less. When the upper limit of the inside diameter of the communication paths 213 falls within the range described above, the capsule-form structures 21 are in sufficient contact with body fluid in an implant part and maintains oxygen supply to the lumens 41. In the case of forming the capsule-form structures 21 and the communication paths 213 with the same oxygen-permeable membrane, the outside diameter of the communication paths 213 is "2L + (10 to 30)" µm wherein L (µm) represents the thickness of the oxygen-permeable membrane.

Here, the mode of forming depressions in both the oxygen-permeable membranes 51 and 61 at once using the resin mold K3 and thereby eliminating the need of aligning the depressions at the time of formation of the capsule-form structures 21 is described. However, the concave surfaces of the separately molded oxygen-permeable membranes 51 and 61 may be laminated except for the moiety of the communication paths 213 such that the depressions of the oxygen-permeable membranes 51 and 61 are aligned with each other, as described in the production method I mentioned above, to form capsule-form structures 21, as a matter of course.

### (II-3) Cell introduction step

In this step, cells C are introduced to the capsule-form structures 21 (see Figure D).

The introduction of the cells C can adopt a method which involves inserting inlet tube 71, injecting a cell suspension, and filling the lumens 41 of the capsule-form structures 21 with the cell suspension via the communication paths 213. An excess of the cell suspension is eventually discharged to the outside of the device from outlet tube 81. After the introduction of the cells C, the inlet tube 71 and the outlet tube 81 may be removed.

### (II-4) Cell proliferation step

In this step, the cells C are allowed to proliferate in the lumens 41 of the capsule-form structures 21, if necessary. This step can be performed in the same way as in the cell proliferation step (1-4) of the production method I.

### (3) Production method III

A third embodiment of the method for producing a cell-encapsulating device according to the present invention comprises the following steps (III-1), (III-2) and (III-3):
(III-1) arraying, in two-dimensional directions in the same plane, a plurality of capsule-form structures in which at least a part of outer shells of the capsule-form structures is formed from an oxygen-permeable membrane, and cells are encapsulated in the inside of the capsule-form structures;
(III-2) contacting the plurality of capsule-form structures thus arrayed with a base material coated with an oxygen-permeable membrane-forming solution; and
(III-3) solidifying the oxygen-permeable membrane-forming solution.

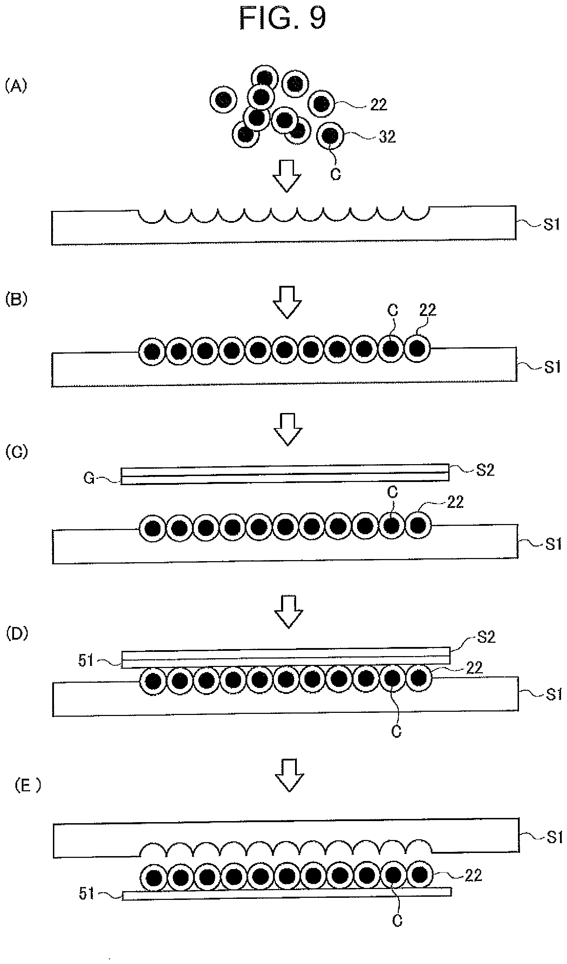

Hereinafter, these steps will be described in order with reference to Figure 9.

### (III-1) Arraying step

In this step, a plurality of capsule-form structures 22 in which at least a part of outer shells 32 is formed from an oxygen-permeable membrane, and cells or cell aggregate C is encapsulated in the inside of the capsule-form structures 22 are arrayed in two-dimensional directions in the same plane.

First, capsule-form structures 22 are prepared according to a known approach (Non Patent Literature 3) (see Figure A).

The outer shells 32 are preferably formed from a hydrogel membrane. Examples of the material thereof include: natural polymers such as agarose, sodium alginate, hyaluronic acid, cellulose and gelatin; and cross-linked forms of synthetic polymers such as polyvinyl alcohol, polyacrylamide, polyacrylic acid, polymethacrylic acid, polyisopropylacrylamide, poly-2-hydroxyethyl methacrylate, poly-2-hydroxyethyl acrylate and polyvinylpyrrolidone with an introduced functional group cross-linkable by light irradiation or the like, and copolymers of each monomer.

The capsule-form structures 22 thus obtained are arranged in two-dimensional directions on guide plate S1 made of a plastic or a metal (see Figure B). In this respect, grooves or hollows can be disposed in the guide plate S1, and the capsule-form structures 22 are arranged so as to fit into the grooves or the hollows.

### (III-2) Base material stacking step

In this step, the plurality of capsule-form structures 22 thus arrayed are contacted with base material S2 coated with oxygen-permeable membrane-forming solution G.

First, base material S2 surface-coated with oxygen-permeable membrane-forming solution G is provided (see Figure C).

The base material S2 is not limited by its material or thickness, and a nylon membrane, a polyester membrane, a polyimide membrane, or the like can be used.

The same material as that of the outer shells 32 of the capsule-form structures 22 is preferably used in the oxygen-permeable membrane-forming solution G. Specifically, an agarose gel solution is preferably used for outer shells 32 made of agarose gel, and a sodium alginate solution is preferably used for outer shells 32 made of alginic acid gel.

Next, the base material S2 is stacked on the capsule-form structures 22 arrayed on the base material S2 such that the surface coated with the oxygen-permeable membrane-forming solution G is kept down (see Figure D). The capsule-form structures 22 may be arranged directly on base material S2 surface-coated with oxygen-permeable membrane-forming solution G without the use of the guide plate S1.

### (III-3) Solidification step

In this step, the oxygen-permeable membrane-forming solution G is solidified. The oxygen-permeable membrane-forming solution G is solidified into oxygen-permeable membrane 51, which is then fixed to the capsule-form structures 22. After the solidification, the oxygen-permeable membrane 51 is separated, together with the capsule-form structures 22, from the guide plate S1, if necessary (see Figure E). In the case of arranging the capsule-form structures 22 directly on base material S2 surface-coated with gel solution G, the separation from the guide plate S1 is unnecessary.

The solidification of the oxygen-permeable membrane-forming solution G can be performed, for example, by decrease in temperature for the agarose gel solution, dipping in a calcium chloride or barium chloride solution for the alginic acid gel solution, or light irradiation for polyvinyl alcohol or the like with an introduced functional group cross-linkable by light irradiation or the like.

Finally, the base material S2 can be peeled off from the oxygen-permeable membrane 51 to obtain a cell-encapsulating device.

### (4) Production method IV

A fourth embodiment of the method for producing a cell-encapsulating device according to the present invention comprises the following steps (IV-1), (IV-3) and (IV-4) and optionally further comprises a step (IV-2) :
(IV-1) receiving cells into a plurality of depressions arrayed in two-dimensional directions on a substrate and isolated from each other with partition walls;
(IV-2) allowing the cells to proliferate in the depressions;
(IV-3) introducing an oxygen-permeable membrane-forming solution onto the depression-formed surface of the substrate; and
(IV-4) solidifying the oxygen-permeable membrane-forming solution, and separating an oxygen-permeable membrane, together with the cells, from the substrate.

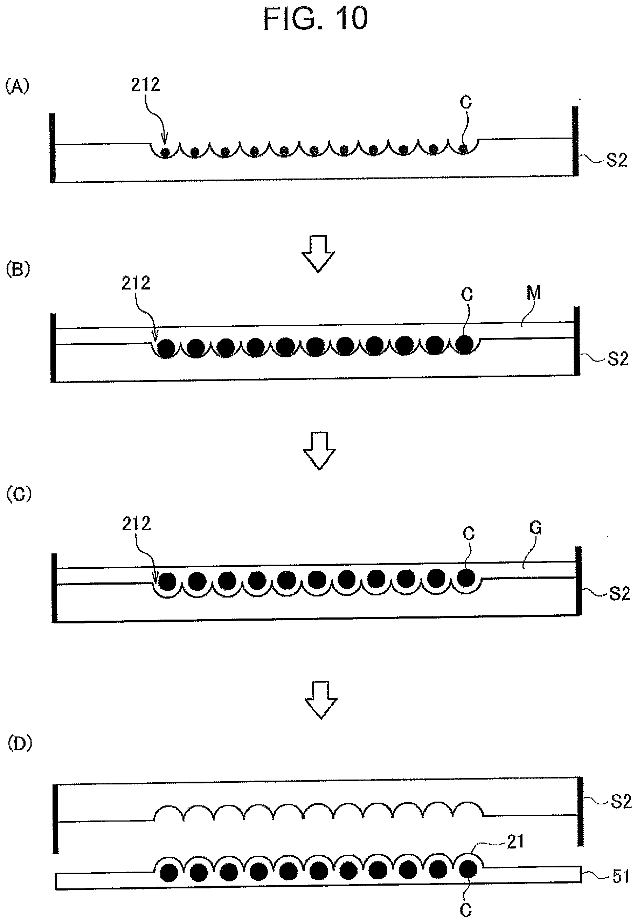

Hereinafter, these steps will be described in order with reference to Figure 10.

### (IV-1) Cell receiving step

In this step, cells C are received into a plurality of depressions 212 arrayed in two-dimensional directions on guide plate S2 and isolated from each other with partition walls (see Figure A).

The guide plate S2 is a plastic or metal substrate provided with depressions 211 capable of receiving cells C. For the guide plate S2, it is preferred that the cells C received in the depressions 212 should be subjected directly to the next cell proliferation step. For example, a general-purpose U-bottomed petri dish can be used.

The depressions 212 have a shape appropriate for the outer shells of capsule-form structures 21 (see Figure D), and the size thereof may be appropriately set in consideration of the outside diameter of the capsule-form structures 21.

### (IV-2) Cell proliferation step

In this step, the cells C are allowed to proliferate in the depressions 212, if necessary (see Figure B).

The proliferation of the cells C can be performed, for example, by filling the depressions 212 with culture medium M and then placing the guide plate S2 in an atmosphere suitable for cell proliferation. The proliferation of the cells forms cell aggregates of the cells C in the depressions 211. The largest diameter of the obtained cell aggregates corresponds to the diameter of the lumens of the capsule-form structures 21 of the resulting cell-encapsulating device.

In this step, the cells C before proliferation or after proliferation may be further induced to differentiate, if necessary. A specific method for the induction of differentiation is as mentioned above.

### (IV-3) Membrane-forming solution introduction step

In this step, oxygen-permeable membrane-forming solution G is introduced onto the depression 212-formed surface of the guide plate S2 (see Figure C). The culture medium M used in the preceding step is replaced with the oxygen-permeable membrane-forming solution G.

The same material as that of the outer shells of the capsule-form structures 21 is preferably used in the oxygen-permeable membrane-forming solution G. Specifically, an agarose gel solution is preferably used for outer shells made of agarose gel, and a sodium alginate solution is preferably used for outer shells made of alginic acid gel.

The specific gravity of the oxygen-permeable membrane-forming solution G is determined according to the specific gravity of the cells C or an aggregated mass thereof. Specifically, the specific gravity of the oxygen-permeable membrane-forming solution G is adjusted to be substantially the same as that of the cell aggregate such that the cell aggregate can float in the introduced oxygen-permeable membrane-forming solution G. It is preferred to impregnate the guide plate S2 therewith and promote the floating of the cell aggregate in the oxygen-permeable membrane-forming solution G.

The amount of the oxygen-permeable membrane-forming solution G introduced may be appropriately set in consideration of the outside diameter of the capsule-form structures 21, and the thickness of the oxygen-permeable membrane 51 (which corresponds to a partial thickness of the outer shells of capsule-form structures 21) to be obtained by solidifying the oxygen-permeable membrane-forming solution G.

### (IV-4) Membrane solution solidification and separation step

In this step, the oxygen-permeable membrane-forming solution G is solidified, and oxygen-permeable membrane 51 is separated, together with the cells C, from the guide plate S2 (see Figure D).

The solidification of the oxygen-permeable membrane-forming solution G can be performed, for example, by decrease in temperature for the agarose gel solution, dipping in a calcium chloride or barium chloride solution for the alginic acid gel solution, or light irradiation for polyvinyl alcohol or the like with an introduced functional group cross-linkable by light irradiation or the like.

Finally, the oxygen-permeable membrane 51 is separated from the guide plate S2 to obtain a cell-encapsulating device comprising capsule-form structures 21 arrayed in two-dimensional directions in the same plane.

The methods I to IV for producing a cell-encapsulating device according to the present invention described above are free from a gap (dead space) between a cell aggregate encapsulated in the inside of the capsule-form structures and the oxygen-permeable membrane. Thus, the methods may supply sufficient oxygen to cells without the controversial inhibition of oxygen supply to cells by the gap, if any.

### Reference Signs List

- 11:: cell-encapsulating device
- 21, 22:: capsule-form structure
- 211, 212:: depression
- 213:: communication path
- 31, 32:: outer shell
- 41:: lumen
- 51, 61:: oxygen-permeable membrane
- 71:: inlet tube
- 81:: outlet tube
- C:: cell
- G:: oxygen-permeable membrane-forming solution
- K1:: male mold
- K2:: female mold
- K3:: resin mold
- M:: culture medium
- S1, S2:: guide plate
- S2:: base material

## Claims

1. A cell-encapsulating device comprising a plurality of capsule-form structures arrayed in two-dimensional directions in the same plane, wherein at least a part of outer shells of the capsule-form structures is formed from an oxygen-permeable membrane, and cells are encapsulated in the inside of the capsule-form structures.

2. A method for producing a cell-encapsulating device, comprising the steps of:
forming a plurality of depressions arrayed in two-dimensional directions on oxygen-permeable membranes;
partially sealing the depressions to form capsule-form structures arrayed in two-dimensional directions in the same plane in which lumens of the capsule-form structures communicate with each other; and
introducing cells to the capsule-form structures.

3. A method for producing a cell-encapsulating device, comprising the steps of:
forming a plurality of depressions arrayed in two-dimensional directions on oxygen-permeable membranes;
introducing cells to the depressions; and
laminating the concave surfaces of the oxygen-permeable membranes such that the depressions of the oxygen-permeable membranes are aligned with each other to form capsule-form structures arrayed in two-dimensional directions in the same plane.

4. A method for producing a cell-encapsulating device, comprising the steps of:
arraying, in two-dimensional directions in the same plane, a plurality of capsule-form structures in which at least a part of outer shells of the capsule-form structures is formed from an oxygen-permeable membrane, and cells are encapsulated in the inside of the capsule-form structures;
contacting the plurality of capsule-form structures thus arrayed with a base material coated with an oxygen-permeable membrane-forming solution; and
solidifying the oxygen-permeable membrane-forming solution.

5. A method for producing a cell-encapsulating device, comprising the steps of:
receiving cells into a plurality of depressions arrayed in two-dimensional directions on a substrate and isolated from each other with partition walls;
introducing an oxygen-permeable membrane-forming solution onto the depression-formed surface of the substrate; and
solidifying the oxygen-permeable membrane-forming solution, and separating an oxygen-permeable membrane, together with the cells, from the substrate.
